# EUROPÄISCHE PATENTANMELDUNG

(11) **EP 1 069 097 A2**
(43) Veröffentlichungstag der Anmeldung: **17.01.2001**
(21) Anmeldenummer: 00112979.0
(22) Anmeldetag: 21.06.2000
(51) Int. Cl.: C07B 53/00, C07D 307/33

(54) **Verfahren zur selektiven Reduktion**

(30) Priorität: 16.07.1999 AT 123699
(71) Anmelder: DSM Fine Chemicals Austria GmbH, 4021 Linz (AT)
(72) Erfinder: Johnson, Dean Vincent, Hertfordshire AL 74 EE (GB); Pöchlauer, Peter, 4040 Linz (AT); Griengl, Herfried, 8047 Graz (AT)
(74) Vertreter: Klostermann, Ingrid

(57) **Zusammenfassung**

Verfahren zur stereoselektiven Reduktion von chiralen α- oder β-Ketoestern, die in γ-Position ein chirales Zentrum besitzen, in einem inerten Lösungsmittel bei Temperaturen von - 80 bis +50°C mit einem Reduktionsmittel, erhalten durch Reaktion von NaBH₄ und (*D*)- oder (*L*)-Weinsäure, zu den jeweils entsprechenden diastereomeren Hydroxyverbindungen.

## Beschreibung

Chiral funktionalisierte Alkohole sind sehr wichtige synthetische Zwischenprodukte, beispielsweise für potentielle Enzymhemmer für die Anti-Krebstherapie, wie etwa Protein-Kinase C - Inhibitoren oder für Anti-HIV-Mittel.
Aus diesem Grund wurden bereits viele Versuche unternommen, diese Verbindungen aus prochiralen Ketonen zu gewinnen.
Eine der gebräuchlichsten Methoden zur Herstellung von optisch aktiven α-Hydroxyestern ist die asymmetrische Reduktion der korrespondierenden α-Ketoester. So wurde bereits die asymmetrische Reduktion von prochiralen Ketonen mit chiralen, von NaBH₄ abgeleiteten Systemen, die durch Reaktion von NaBH₄ mit beispielsweise Monosacchariden oder α-Aminosäurederivaten, insbesondere L-Prolinderivaten, erhalten werden, genauer untersucht. Diese Methode liefert jedoch, wie aus Bull. Chem. Soc. Jpn., 64, 175-182 (1991) bekannt ist, nur mäßige optische Reinheiten. Aus diesem Grund wurden in dieser Literatur andere chirale, von NaBH₄ abgeleitete Systeme untersucht. So wurde Ethyl-2-(3,4-Isopropylidendioxyphenyl)-2-oxoacetat mit einem System, erhalten durch Umsetzung von NaBH₄ mit (*R,R*)-Weinsäure, zu der entsprechenden (*R*)-Hydroxyverbindung reduziert, wobei ein ee von bis zu 78% erzielt wurde. Im Gegensatz dazu ergab die asymmetrische Reduktion von Propiophenon mit dem Umsetzungsprodukt aus NaBH₄ und (*S,S*)-Weinsäure (S)-1-Phenyl-1-propanol in einer optischen Ausbeute von lediglich 10%.
In EP-A1-0 320 096 ist ebenfalls die Reduktion von prochiralen Ketoestern oder Ketonen mittels NaBH₄ und Weinsäure beschrieben. In den Beispielen wird dabei gezeigt, dass die Verwendung von (*R,R*)-Weinsäure die entsprechenden (*R*)-Hydroxyverbindungen in einer optischen Ausbeute von lediglich 15 bis maximal 85% liefert.
In Synthetic Communications, 14(10), 955-959 (1984) wurde die Effizienz des NaBH₄/Weinsäuresystems bei der Reduktion von cyclischen Ketonen untersucht. Dabei wurden Ketone, wie 2-Methyl-, 3-Methyl-, 4-Methyl- und 4-t-Butylcyclohexanon zu den entsprechenden Alkoholen reduziert, wobei mit dem NaBH₄/Weinsäuresystem deutlich höhere Anteile an den stabileren äquatorialen Alkoholen als mit NaBH₄ alleine erzielt wurden. Eine Veränderung der optischen Aktivität der Weinsäure hatte keinen erkennbaren Einfluß auf das stereochemische Ergebnis der Reduktion.
Vielmehr läßt sich aus der Literatur ableiten, dass die Stereoselektivität stark von der Struktur des Substrates abhängig ist und dass im Substrat vorhandene chirale Zentren, beziehungsweise die Art und Größe der Substituenten des Substrates einen starken Einfluß auf den Verlauf der Reduktion haben. Dies wird beispielsweise durch J. Org. Chem. 1999, 64, 2172-2173 bestätigt. In dieser Literatur wurde die Stereoselektivität bei der Reduktion von β-Hydroxy oder β-Alkoxyketonen mittels Sml₂ untersucht, wobei festgestellt wurde, dass Substrate mit dem β-Hydroxysubstituenten in hoher Ausbeute und guter optischer Reinheit reduziert werden konnten, wobei die Hydroxygruppe sowohl die Stereoselektivität, als auch die Reaktionsgeschwindigkeit wesentlich bestimmte. Substrate, die eine geschützte β-Hydroxygruppe, etwa eine β-OBn oder OTBS-Gruppe aufwiesen, waren im Vergleich zu Verbindungen mit der freien Hydroxyguppe fast zur Gänze inert. Bei diesen Substraten wurde keine Reduktion erzielt. Auch aus J. Org. Chem., Vol. 56, No. 24, 1991 geht hervor, dass die Größe der Substituenten einen großen Einfluss auf die Stereoselektivität hat.

Unerwarteterweise wurde nun gefunden, dass α- oder β-Ketoester, die in γ-Position ein chirales Zentrum besitzen, mit NaBH₄ und (*D*)- oder (*L*)-Weinsäure zu den jeweils korrespondierenden diastereomeren Hydroxyestern in hoher optischer Reinheit und hoher Ausbeute reduziert werden können, wobei das in der Ausgangsverbindung vorhandene chirale Zentrum keinen Einfluss auf die Bildung der Diastereomeren hat.

Gegenstand der Erfindung ist demnach ein Verfahren zur stereoselektiven Reduktion von chiralen α- oder β-Ketoestern, das dadurch gekennzeichnet ist, dass α- oder β-Ketoester, die in γ-Position ein chirales Zentrum besitzen, in einem inerten Lösungsmittel bei Temperaturen von - 80 bis +50°C mit einem Reduktionsmittel, erhalten durch Reaktion von NaBH₄ und (*D*)- oder (*L*)-Weinsäure, zu den jeweils entsprechenden diastereomeren Hydroxyverbindungen reduziert werden.

Bei dem erfindungsgemäßen Verfahren werden chirale α- oder β-Ketoester, die in γ-Position ein chirales Zentrum besitzen, reduziert. Geeignete Substrate weisen demnach bevorzugt eine offenkettige, gegebenenfalls verzweigte Alkyl- oder Alkenylkette auf. Die Alkyl- oder Alkenylkette besteht dabei aus 4 bis 30, bevorzugt 4 bis 15 C-Atomen. Im Falle einer Alkenylkette, kann diese 1 bis 3 Doppelbindungen aufweisen. Die Kette ist in Position 1 mit einer Carboxylatgruppe substituiert. Die Estergruppe leitet sich dabei von einem primären, sekundären oder tertiärem Alkohol ab. Bevorzugt sind Ester von primären Alkoholen. Dies sind demnach C₁-C₂₀, bevorzugt C₁-C₆-Alkylester, wie Methyl-, Ethyl-, Propyl-, Butyl-, Hexylester, u.s.w.. Entweder in α- oder β-Position zum Carboxylat, bevorzugt in α-Position, befindet sich eine Ketogruppe. In γ-Position weisen die Substrate ein chirales Zentrum auf. Bevorzugt befindet sich in γ-Position eine geschützte OH-Gruppe, beispielsweise eine t-Butyldiphenylsilyloxy-Gruppe, eine Trimethylsilyloxy-Gruppe, eine Benzyloxy-Gruppe oder eine andere gebräuchliche Schutzgruppe, als Substituent.
Besonders bevorzugt sind Substrate, die sich aus ungesättigten Cyanhydrinen mittels Blaise-Reaktion herstellen lassen. Ganz besonders bevorzugt ist (4S)-Methyl-3-oxo-4-tert-butylsilyloxy-undec-5-enoat.

Das entsprechende Substrat wird erfindungsgemäß mit einem Reduktionsmittel, das durch Umsetzung von NaBH₄ mit (*D*)- oder (*L*)-Weinsäure erhalten wird, reduziert.
Je nachdem, welches Diastereomer der entsprechenden Hydroxyverbindung gewünscht wird, wird dabei NaBH₄ entweder mit (*D*)- oder mit (*L*)-Weinsäure umgesetzt. Die Herstellung des eigentlichen Reduktionsmittels, (das von (*D*)- oder (*L*)-Weinsäure abgeleiteten Natriumacyloxyborhydrid) erfolgt bevorzugt in situ, wie beispielsweise in Synthetic Communications, 14(10), 955-959 (1984); Bull. Chem. Soc. Jpn., 64, 175-182 (1991) oder J. Chem. Soc. Perkin Trans I, 1827 (1990) u.s.w. beschrieben. Bevorzugt wird dabei die entsprechende Weinsäure und NaBH₄ in einem geeigneten inerten Verdünnungsmittel, das bevorzugt auch als Lösungsmittel für die Reduktion dient, suspendiert, wobei NaBH₄ wiederum bevorzugt portionsweise zu einer Lösung der Weinsäure zugesetzt und die so erhaltene Suspension 0,5 bis 6 Stunden bis zur Rückflußtemperatur erhitzt wird. Geeignete Verdünnungs- bzw. Lösungsmittel sind beispielsweise Alkohole, wie etwa 2-Propanol, t-Butanol, u.s.w., Ether, wie Tetrahydrofuran (THF), Dioxan, Diethylether u.s.w., Aromaten, wie Benzol, Toluol, Xylol, u.s.w. oder andere unter den Reaktionsbedingungen inerte Lösungsmittel. Gegebenenfalls können obige Lösungsmittel auch als Lösungsmittelgemisch eingesetzt werden. Bevorzugt wird THF oder 2-Propanol eingesetzt.
Das molare Verhältnis von NaBH₄ zu (*D*)- bzw. (*L*)-Weinsäure liegt zwischen 1:0,5 bis 1:1,5, bevorzugt beträgt das molare Verhältnis 1:1.
Anschließend an das Erwärmen der Suspension wird auf bis zu - 50°C, bevorzugt auf - 30°C und besonders bevorzugt auf -20°C, abgekühlt. In die so erhaltene Lösung des Reduktionsmittels wird sodann eine Lösung des zu reduzierenden Substrates eingebracht. Als Lösungsmittel eignen sich wiederum die oben aufgelisteten Lösungsmittel. Bevorzugt wird bei der in situ-Herstellung des Reduktionsmittels und bei der Reduktion das gleiche Lösungsmittel eingesetzt. Bevorzugt wird die Substratlösung in mehreren Portionen, besonders bevorzugt tropfenweise zugegeben, sodass die Temperatur konstant auf besonders bevorzugt -20°C gehalten wird.
Das Reduktionsmittel liegt dabei im Überschuss zum Substrat vor. Bevorzugt ist ein molares Verhältnis von Substrat zu Reduktionsmittel von 1:2 bis 1:6, besonders bevorzugt von etwa 1:4.
Das Reaktionsgemisch wird dabei gerührt. Nach vollendeter Reduktion, d.h. in Abhängigkeit vom gewählten Substrat nach 1 bis 60 Stunden wird dem Reaktionsgemisch zur Isolierung des entsprechenden Diastereomeren, beziehungsweise zum Abbrechen der Reaktion beispielsweise Salzsäure oder Schwefelsäure zugesetzt. Bevorzugt wird weiters Ethylacetat oder andere geeignete Lösungsmittel, sowie gegebenenfalls Natriumchlorid bis zum Erreichen des Sättigungspunktes zugegeben. Nach erfolgter Phasentrennung wird das Endprodukt aus der organischen Phase extrahiert und gereinigt.

Durch das erfindungsgemäße Verfahren ist es unerwarteterweise möglich durch die Auswahl von (*D*)- oder (*L*)-Weinsäure, die entsprechende diastereomere Hydroxyverbindung in hoher Ausbeute und optischer Reinheit zu erhalten. Dies ist insbesondere bei der Reduktion von (4S)-Methyl-3-oxo-4-tert-butylsilyloxy-undec-5-enoat von besonderem Vorteil, da mit dem erfindungsgemäßen Verfahren gezielt) je nachdem ob (*D*)- oder (*L*)-Weinsäure eingesetzt wird, entweder (3*R*, 4*S*)- oder (3*S*, 4*S*)- Methyl-3-hydroxy-4-tert-butylsilyloxy-undec-5-enoat in ausgezeichnetem Diastereomerenüberschuss von über 90% hergestellt werden kann. Diese Verbindungen sind wertvolle Zwischenprodukte für die Herstellung eines Inhibitors für Protein-Kinase C bzw. für L-AZT (Azidothymidin), einem potentiellen Anti-HIV-Mittel.
Ein weiterer Gegenstand ist demnach die Verwendung von erfindungsgemäß hergestellten (3*R*, 4*S*)- oder (3*S*, 4*S*)- Methyl-3-hydroxy-4-tert-butylsilyloxy-undec-5-enoat zur Herstellung der entsprechenden enantiomeren- und diastereomerenreinen Lactone (4*R*, 5*S*)-5-Hydroxymethyl-4-tetradecanoyl-tetrahydro-furan-2-on und (4*S*, 5*S*)-5-(E-Hept-1-enyl)-4-hydroxy-tetrahydro-furan-2-on, sowie dessen weitere Umsetzung zu dem L-AZT-Intermediat (2*R*, 4*S*, 5*S*)-5-Acetoxy-3-azido-2-hydroxymethyl-oxolan.
Die weitere Umsetzung von (3*R*, 4*S*)- Methyl-3-hydroxy-4-tert-butylsilyloxy-undec-5-enoat zu (4*R*, 5*S*)-5-Hydroxymethyl-4-tetradecanoyl-tetrahydro-furan-2-on, einem Inhibitor für Protein-Kinase C, weist eine Gesamtausbeute von 32% auf, während bisher bekannte Herstellungsvarianten, wie etwa in J.Am.Chem.Soc., **1992**, 114, 1059-1070 beschrieben, zu Ausbeuten von lediglich 8% führen. Weiters benötigt die erfindungsgemäße Weiterverarbeitung lediglich 7 Stufen, währen bisher 14 Stufen üblich waren.
Die erfindungsgemäße Verwendung von (3*R*, 4*S*)- Methyl-3-hydroxy-4-tert-butylsilyloxy-undec-5-enoat (A) zur Herstellung von (4*R*, 5*S*)-5-Hydroxymethyl-4-tetradecanoyl-tetrahydro-furan-2-on (E) beinhaltet demnach folgende Schritte:
a) Umsetzung mit Tetrabutylammoniumfluorid (TBAF) in einem unter den Reaktionsbedingungen inerten Lösungsmittel, wie etwa THF bei 10 bis 40°C, bevorzugt bei 20-30°C und Isolierung des Zwischenproduktes (4*R*, 5*S*)-5-(*E*-Hept-1-enyl)-4-hydroxy-tetrahydro-furan-2-on (C) durch Extraktion, sodann
b) Umsetzung mit einem Gemisch aus Pyridin und Myristoylchlorid in CH₂Cl₂ bei 10 bis 40°C, bevorzugt bei 20-30°C und Isolierung des Zwischenproduktes (4*R*, 5*S*)-5-(*E*-Hept-1-enyl)-4-tetradecanoyl-tetrahydro-furan-2-on (D) durch Extraktion, und anschließend
c) Umsetzung mit Ozon bei -80 bis - 60°C, erwärmen auf 10 bis 40°C, bevorzugt bei 20-30°C und Isolierung des Endproduktes (4*R*, 5*S*)-5-Hydroxymethyl-4-tetradecanoyl-tetrahydro-furan-2-on (E) durch Zusatz von BH₃:Me₂S unter Edelgasatmosphäre , sowie von MeOH nach 10 bis 30h.

Die Zwischenverbindung für L-AZT wird durch die erfindungsgemäße Verwendung von (3*S*, 4*S*)- Methyl-3-hydroxy-4-tert-butylsilyloxy-undec-5-enoat (B) durch folgende Schritte erhalten:
a) Umsetzung mit TBAF in einem unter den Reaktionsbedingungen inerten Lösungsmittel, wie etwa THF bei 10 bis 40°C, bevorzugt bei 20-30°C und Isolierung des Zwischenproduktes (4*S*, 5*S*)-5-(*E*-Hept-1-enyl)-4-hydroxy-tetrahydro-furan-2-on (F), sodann
b) Umsetzung mit einer gekühlten (0 bis 10°C, bevorzugt 5°C) Lösung von Imidazol in DMF und t-Butyl-diphenylsilylchlorid, erwärmen auf 10 bis 40°C, bevorzugt auf 20-30°C und Isolierung des Zwischenproduktes (4*S*, 5*S*)- 4-*tert*-butyldiphenylsilyloxy-5-(*E*-Hept-1-enyl)-tetrahydro-furan-2-on (G) durch Extraktion, weiters
c) Umsetzung mit Di-iso-butyl-aluminiumhydrid (DIBAL) unter Edelgasatmosphäre bei -80 bis -60°C, erwärmen auf 10 bis 40°C, bevorzugt auf 20-30°C und Isolierung des Zwischenproduktes (2*S*, 4*S*, 5*S*)-5-Acetoxy-3-*tert*-butyldiphenylsilyloxy-2-(*E*-hept-1-enyl)-oxolan (H) durch Extraktion, weiters
d) Umsetzung einer gekühlten Lösung (0 bis 10°C, bevorzugt 5°C) von ((2*S*, 4*S*, 5*S*)-5-Acetoxy-3-*tert*-butyldiphenylsilyloxy-2-(*E*-hept-1-enyl)-oxolan mit TBAF, erwärmen auf 10 bis 40°C, bevorzugt auf 20-30°C und Isolierung des Zwischenproduktes (2*S*, 4*S*, 5*S*)-5-Acetoxy-2-(*E*-hept-1-enyl)-3-hydroxy-oxolan (I) durch Extraktion, sowie
e) Umsetzung einer gekühlten Lösung (0 bis 10°C, bevorzugt 5°C) von ((2*S*, 4*S*, 5*S*)-5-Acetoxy-2-(*E*-hept-1-enyl)-3-hydroxy-oxolan mit Pyridin und mit Trifluoressigsäureanhydrid. Zugabe von Natriumazid, gefolgt von DMF, erwarmen auf 10 bis 40°C, bevorzugt auf 20-30°C und Isolierung des Zwischenproduktes (2*S*, 4*S*, 5*S*)-5-Acetoxy-3-azido-2-(*E*-hept-1-enyl)-oxolan (J) durch Extraktion und
f) Umsetzung mit Ozon, Reduktion analog zu Hoffman, J. Org. Chem. 1997 (62), 2458-2465 oder Hudlicky, , J. Org. Chem. 1998 (63) 510-520 und Isolierung des Endproduktes (2*R*, 4*S*, 5*S*)-5-Acetoxy-3-azido-2-hydroxymethyl-oxolan (K).

### Beispiel 1:

### (3R, 4S)- Methyl-3-hydroxy-4-tert-butylsilyloxy-undec-5-enoat (A)

Zu einer Lösung von (*D*)-Weinsäure (9.6 g, 64 mmol) in trockenem THF (150 ml) wurde NaBH₄ (2.42 g, 64 mmol) portionsweise zugegeben, die so erhaltene Suspension 2,5 h auf Rückflusstemperatur erhitzt und anschließend auf -20°C abgekühlt. Danach wurde eine Lösung von (4*S*)- Methyl-3-oxo-4-tert-butylsilyloxy-undec-5-enoat (7.54g, 16 mmol) in trockenem THF (75 ml) tropfenweise zugesetzt, wobei die Temperatur auf -20°C blieb. Nach 40 h wurde dem Reaktionsgemisch Salzsäure (1M, 150 ml) zugegeben, wodurch sich die Temperatur auf Raumtemperatur erhöhte. Es wurde weitere 15 Minuten gerührt. Sodann wurde Ethylacetat (500 ml), gefolgt von festem Natriumchlorid bis zum Erreichen des Sättigungspunktes eingebracht. Die wässrige Phase wurde abgetrennt und mit weiteren 200 ml Ethylacetat extrahiert. Die organischen Phasen wurden vereint, zweimal mit je 150 ml gesättigter Natriumbicarbonatlösung und anschließend mit 100 ml einer gesättigten Natriumchloridlösung gewaschen, über Natriumsulfat getrocknet, filtriert und unter reduziertem Druck konzentriert. Das Rohprodukt wurde durch Säulenchromatographie (90:10 bis 80:20, Petrolether: Ethylacetat) gereinigt, wodurch (3*R*, 4*S*)- Methyl-3-hydroxy-4-tert-butylsilyloxy-undec-5-enoat als farbloses Öl in einer Ausbeute von 93% (6.97g, 14.7 mmol) erhalten wurde.
d.e.= 92% (chiral HPLC, OD-H, 99.75:0.25 Heptan: Isopropanol, 0.6 ml/min, 254 nm); ¹H NMR (CDCl₃, 200MHz): δ (ppm) 0.88 (t, 3H), 1.07 (s, 9H), 1.13- 1.30 (m, 3H), 1.74-1.84 (m, 2H), 2.44 (d, 1H, *J* = 2.38 Hz), 2.47 (s, 1H), 2.62 (d, 1H, *J* = 3.47 Hz), 3.67 (s, 3H), 4.01-4.12 (m, 2H), 5.14-5.44 (m, 2H), 7.30-7.49 (m, 6H), 7.62-7.73 (m, 4H), ¹³C NMR (CDCl₃, 200MHz): δ (ppm), 14.06,19.41, 22.51, 27.11, 28.45, 31.37, 32.14, 37.07, 51.76, 71.67, 77.61, 127.43, 127.69, 129.66, 129.84, 133.57, 133.81, 135.71, 135.95, 136.08, 172.59.

### Beispiel 2:

### (3S, 4S)- Methyl-3-hydroxy-4-tert-butylsilyloxy-undec-5-enoat (B)

Zu einer Lösung von (*L*)-Weinsäure (8.06g, 53.72 mmol) in trockenem THF (125 ml) wurde NaBH₄ (2.03g, 53.72 mmol) portionsweise zugegeben, die so erhaltene Suspension 2,75h auf Rückflusstemperatur erhitzt und anschließend auf -20°C abgekühlt. Danach wurde eine Lösung von (4*S*)- Methyl-3-oxo-4-tert-butylsilyloxy-undec-5-enoat (5.38g, 11.55 mmol) in trockenem THF (75 ml) tropfenweise zugesetzt, wobei die Temperatur auf -20°C blieb. Nach 43h wurde dem Reaktionsgemisch Salzsäure (1M, 125 ml) zugegeben, wodurch sich die Temperatur auf Raumtemperatur erhöhte. Es wurde weitere 15 Minuten gerührt. Sodann wurde Ethylacetat (500 ml), gefolgt von festem Natriumchlorid bis zum Erreichen des Sättigungspunktes eingebracht. Die wässrige Phase wurde abgetrennt und mit weiteren 200 ml Ethylacetat extrahiert. Die organischen Phasen wurden vereint, zweimal mit je 150 ml gesättigter Natriumbicarbonatlösung und anschließend mit 100 ml einer gesättigten Natriumchloridlösung gewaschen, über Natriumsulfat getrocknet, filtriert und unter reduziertem Druck konzentriert. Das Rohprodukt wurde durch Säulenchromatographie (90:10 bis 80:20, Petrolether: Ethylacetat) gereinigt, wodurch (3*R*, 4*S*)- Methyl-3-hydroxy-4-tert-butylsilyloxy-undec-5-enoat als farbloses Öl in einer Ausbeute von 89% (4.77g, 10.20 mmol) erhalten wurde.
d.e.= 92% (chiral HPLC, OD-H, 99.75:0.25 Heptan: Isopropanol, 0.6 ml/min, 254 nm); ¹H NMR (CDCl₃, 200MHz): δ (ppm) 0.88 (t, 3H), 1.07 (s, 9H), 1.13- 1.30 (m, 3H), 1.77-1.86 (m, 2H), 2.36 (dd, 1H, *J* = 15.63, 8.60 Hz), 2.53 (dd, 1H, *J* = 15.87, 3.23 Hz), 2.76 (d, 1H, *J* = 3.85 Hz),3.68 (s, 3H), 3.97-4.08 (m, 2H), 5.16-5.41 (m, 2H), 7.31-7.49 (m, 6H), 7.63-7.71 (m, 4H), ¹³C NMR (CDCl₃, 200MHz): δ (ppm), 14.06,19.43, 22.51, 27.11, 28.37, 31.37, 32.14, 37.39, 51.78, 71.74, 77.71, 127.43, 127.71, 129.67, 129.85, 133.51, 133.70, 135.81, 135.92, 136.07, 172.66.

### Beispiel 3:

### Herstellung (4R, 5S)-5-Hydroxymethyl-4-tetradecanoyl-tetrahydro-furan-2-on (E) aus (3R, 4S)- Methyl-3-hydroxy-4-tert-butylsilyloxy-undec-5-enoat (A)

a) Zu einer gekühlten (5°C) Lösung von A (6.47 g, 13.83 mmol), hergestellt analog Beispiel 1, in 165 ml THF wurden 0.691 M TBAF in 30.0 ml Acetonitril (20.74 mmol) tropfenweise zugegeben. Nach 48 Stunden bei Raumtemperatur wurden 300 ml Ethylacetat zugegeben und die organische Phase mit Salzwasser (3 x 25 ml) gewaschen, getrocknet, filtriert und im Vakuum aufkonzentriert. Der Rückstand wurde mittels Säulenchromatographie gereinigt (70:30 bis 50:50, Petrolether:Ethylacetat). Ausbeute an (4*R*, 5*S*)-5-(*E*-Hept-1-enyl)-4-hydroxy-tetrahydro-furan-2-on (C) als weißer Feststoff: 85% (2.23 g, 11,8 mmol).
   ¹H NMR(CDCl₃, 200MHz): δ(ppm) 0.86 (t, 3H, *J*= 7.08 Hz), 1.21-1.43 (m, 6H), 2.04 (q, 2H, *J* = 6.84 und 6.54), 2.47 (dd, 1H, *J* = 17.82 und 3.66 Hz), 2.78 (dd, 1H, *J* = 17.77 und 6.16 Hz), 3.12 (bs, 1H), 4.30 (m, 1H), 4.79 (dd, 1H, *J* = 6.34 und 2.44 Hz), 5.42 (dd, 1H, *J* = 15.38 und 6.57 Hz), 5.83 (dt, 1H, *J* = 15.38 und7.56 Hz); ¹³C NMR (CDCl₃, 200MHz): δ (ppm), 14.04, 22.48, 28.43, 31.34, 32.23, 37.02, 72.21, 87.98, 124.34, 136.34, 172.59.
b) Zu einer Lösung von C (150 mg; 0.75 mmol) in 1 ml CH₂Cl₂ wurde ein Gemisch aus 90 mg Pyridin (1.13 mmol) und 224 mg Myristoylchlorid (0.90 mmol) in 2 ml CH₂Cl₂ unter Rühren zugegeben. Die so erhaltene Lösung wurde weitere 20 h bei Raumtemperatur gerührt. Anschließend wurde ein Gemisch aus CH₂Cl₂/H₂O (je 15 ml) zugegeben und die organische Phase mit H₂O (2 x 10 ml), CuSO₄ (3% w/v; 10 ml) und Salzwasser (10 ml) gewaschen. Die organische Phase wurde abgetrennt, getrocknet, filtriert und im Vakuum aufkonzentriert. Der Rückstand wurde mittels Säulenchromatographie (95:05, Petrolether: Ethylacetat) gereinigt. Ausbeute an (4*R*, 5*S*)-5-(*E*-Hept-1-enyl )-4-tetradecanoyl-tetrahydro-furan-2-on (D) als weißer Feststoff: 85% (249 mg, 0.51 mmol).
   ¹H NMR (CDCl₃, 200MHz): δ (ppm) 0.86 (t, 3H, *J* = 7.08 Hz), 1.18-1.45 (m und Überlappungen s, 26H), 1.53-1.70 (m, 2H), 2.04 (q, 2H, *J* = 7.82 und 6.10), 2.33 (t, 2H, *J* = 7.33 Hz), 2.52 (dd, 1H, *J* = 18.31 und 1.76 Hz), 2.90 (dd, 1H, *J* = 18.31 und 6.54 Hz), 4.91 (d, 1H, *J* = 5.56 Hz), 5.12 (dt, 1H, *J* = 6.40 und 1.71 Hz), 5.46 (ddt, 1H, *J* = 15.52, 5.70 und 1.33 Hz), 5.83 (dt, 1H, *J* = 15.44 und 6.72 Hz); ¹³C NMR (CDCl₃, 200MHz): δ (ppm), 14.04, 14.17, 22.49, 22.74, 28.39, 29.10, 29.26, 29.40, 29.47, 29.63, 29.68, 31.33, 31.96, 32.18, 33.74, 34.12, 73.62, 84.76, 123.67, 135.98, 172.97, 174.13.
c) Durch eine Lösung von 75 mg D (0.18 mmol) in 9 ml CH₂Cl₂ bei -78°C wurde eine Stunde lang Ozon durchgeleitet. Danach wurde die Lösung auf Raumtemperatur erwärmt, für 15 Minuten gerührt und anschließend unter Argonatmosphäre mit BH₃:Me₂S (1M in CH₂Cl₂; 0.75 ml; 0.75 mmol) tropfenweise innerhalb von 25 Minuten zugesetzt. Nach 20 h wurden 0.5 ml MeOH zugegeben und weitere 2 h gerührt. Das Reaktionsgemisch wurde im Vakuum aufkonzentriert und der Rückstand mittels Säulenchromatographie gereinigt (90:10 bis 65:35, Petrolether:Ethylacetat). Ausbeute an (4*R*, 5*S*)-5-Hydroxymethyl-4-tetradecanoyl-tetrahydro-furan-2-on (E) als weißer Feststoff: 40% (25 mg, 0.07 mmol).
   ¹H NMR (CDCl₃, 200MHz): δ (ppm) 0.88 (t, 3H, *J* = 7.08 Hz), 1.23 (s, 20H), 1.55-1.68 (m, 2H), 2.33 (t, 2H, *J* = 7.32 Hz), 2.55 (dd, 1H, *J* = 18.58 und 1.90 Hz), 3.07 (dd, 1H, *J* = 18.60 und 7.54 Hz), 3.93 (m, 2H), 4.50 (m, 1H), 5.36 (dt, 1H, *J* = 7.43 und 1.71 Hz); ¹³C NMR (CDCl₃, 300MHz): δ (ppm), 14.36, 22.94, 24.98, 29.32, 29.46, 29.59, 29.67, 29.88, 32.17, 34.32, 35.68, 62.54, 71.75, 85.73, 173.74, 175.38.

### Beispiel 4:

### Herstellung von (2R, 4S, 5S)-5-Acetoxy-3-azido-2-hydroxymethyl-oxolan (K) aus (3S, 4S)-Methyl-3-hydroxy-4-tert-butylsilyloxy-undec-5-enoat (B).

a) Zu einer gekühlten (5°C) Lösung von B (4.67 g, 9.97 mmol), hergestellt analog Beispiel 2, in 118 ml THF wurden 0.691 M TBAF in 21.6 ml Acetonitril (20.74 mmol) tropfenweise zugegeben. Nach 20 Stunden bei Raumtemperatur wurden 300 ml Ethylacetat zugegeben und die organische Phase mit Salzwasser (3 x 25 ml) gewaschen, getrocknet, filtriert und im Vakuum aufkonzentriert. Der Rückstand wurde mittels Säulenchromatographie gereinigt (60:40 bis 40:60, Petrolether:Ethylacetat). Ausbeute an (4*S*, 5*S*)-5-(*E*-Hept-1-enyl)-4-hydroxy-tetrahydro-furan-2-on (F) als weißer Feststoff: 90% (1.78 g, 8.98 mmol).
   ¹H NMR (CDCl₃, 200MHz): δ (ppm) 0.88 (t, 3H, *J* = 7.08 Hz), 1.23-1.53 (m, 6H), 2.04 (q, 2H, *J* = 6.83 und 6.73), 2.27 (bs, 1H), 2.59 (dd, 1H, *J* = 17.63 und 1.41 Hz), 2.78 (dd, 1H, *J* = 17.57 und 5.12 Hz), 4.46 (m, 1H), 4.86 (dd, 1H, *J* = 6.84 und 3.18 Hz), 5.58 (dd, 1H, *J* = 15.49 und 6.89 Hz), 5.97 (dt, 1H, *J* = 15.49 und 6.73 Hz); ¹³C NMR (CDCl₃, 200MHz): δ (ppm), 14.06, 22.51, 28.48, 31.39, 32.49, 38.77, 69.77, 84.88, 121.35, 139.03, 175.58.
b) Zu einer gekühlten Lösung (5°C) von 825 mg Imidazol (12.12 mmol) in DMF (0.5 ml) wurden 2.49 g tert. Butyl-diphenylsilylchlorid (9.09 mmol) zugegeben. Nach 15 Minuten wurde die Verbindung F (1.20 g; 6.06 mmol) tropfenweise zugesetzt, das Reaktionsgemisch auf Raumtemperatur erwärmt und 4 H bei dieser Temperatur gerührt. Danach wurden 100 ml Wasser zugegeben und die wässrige Phase mit CH₂Cl₂ (3 x 75 ml9 extrahiert. Die organischen Extrakte wurden kombiniert, getrocknet, filtriert und im Vakuum aufkonzentriert. Der Rückstand wurde mittels Säulenchromatographie gereinigt (90:10 bis 85:15, Petrolether:Ethylacetat). Ausbeute an (4*S*, 5*S*)- 4-tert-butyldiphenylsilyloxy-5-(*E*-Hept-1-enyl)-tetrahydro-furan-2-on (G) als farblose Flüssigkeit: 77% (2.04 g, 4.67 mmol).
   ¹H NMR (CDCl₃, 200MHz): δ (ppm) 0.90 (t, 3H, *J* = 6.51 Hz), 1.08 (s, 9H), 1.22-146 (m, 6H), 2.07-2.16 (m, 2H), 2.29-2.51 (m, 2H), 4.5-4.54 (m, 1H), 4.67-4.72 (m, 1H), 5.82-5.86 (m, 2H), 7.34-7.49 (m, 6H), 7.59-7.65 (m, 4H); ¹³C NMR (CDCl₃, 200MHz): δ (ppm), 14.08, 19.27, 22.58, 26.85, 28.41, 31.50, 32.48, 38.88, 71.63, 85.63, 123.39, 127.85, 127.98, 130.12, 130.22, 132.58, 133.08, 135.78, 135.89, 138.11, 175.25.
c) Die Lösung von G wurde innerhalb von 15 Minuten bei -78°C und unter Argonatmosphäre mit DIBAL (1M in Hexan; 11.6 ml; 11.6 mmol) versetzt. Nach 1.5 h wurden 2 ml Wasser zugesetzt und das Reaktionsgemisch weitere 15 Minuten bei -78°C gerührt, bevor es sich auf Raumtemperatur erwärmte, bei der weitere 1.5 h gerührt wurden. Das Gemisch wurde mit 100 ml Et₂O verdünnt, in eine gesättigte Lösung aus Natriumditartrat (110 ml) gegossen und weiter gerührt. Nach 1 h wurde die wässrige Phase mit Et₂O (3 x 100 ml) extrahiert, die organischen Extrakte kombiniert, getrocknet, filtriert und im Vakuum aufkonzentriert. Der Rückstand wurde in 30 ml CH₂Cl₂ aufgelöst, auf 5°C gekühlt und mit einer Lösung aus Essigsäureanhydrid (2.46 ml; 26.13 mmol) und 4-Dimethylamminopyridin (DMAP) (47 mg; 0.387 mmol) versetzt. Das Gemisch wurde 3.5 h gerührt, wobei es sich auf Raumtemperatur erwärmte. Danach wurde das Gemisch in eine gesättigte NaHCO₃-Lösung (40 ml) gegossen. Die wässrige Phase wurde mit Et₂O (4 x 40 ml) extrahiert, die organischen Extrakte kombiniert, getrocknet, filtriert und im Vakuum aufkonzentriert. Der Rückstand wurde mittels Säulenchromatographie gereinigt (90:10 bis 85:15, Petrolether: Ethylacetat). Ausbeute an (2*S*, 4*S*, 5*S*)-5-Acetoxy-3-*tert*-butyldiphenylsilyloxy-2-(*E*-hept-1-enyl)-oxolan (H) als farbloses Öl: 66% (1.22 g; 2.54 mmol)
   ¹H NMR (CDCl₃, 200MHz): δ (ppm) 0.89 (t, 3H, *J* = 6.59 Hz), 1.07 (s, 9H), 1.25-1.49 (m, 6H), 1.88-2.24 (m und Überlappungen s, 7H), 4.37-4.43 (m, 1H), 4.45-4.52 (m, 1H), 5.74-5.77 (m, 2H), 6.37 (dd, 2H, 7.34-7.49 (m, 6H), 7.59-7.65 (m, 4H); ¹³C NMR (CDCl₃, 200MHz): δ (ppm), 14.07, 19.28, 21.31, 26.90, 28.56, 31.58, 32.51, 42.33, 74.04, 84.54, 97.66, 125.30, 127.76, 127.67, 129.82, 129.89, 133.27, 133.92, 135.84, 135.96, 136.41, 170.36.
d) Zu einer gekühlten (5°C) Lösung von H (140 mg, 0.296 mmol), in 4 ml THF wurde TBAF (1M in THF; 0.30 ml; 0.30 mmol) tropfenweise innerhalb 10 Minuten zugegeben. Nach 1.5 Stunden Rühren bei Raumtemperatur wurde das Reaktionsgemisch in Ethylacetat/Wasser (10 ml/ 5 ml) gegossen und die wässrige Phase mit Ethylacetat (2 x 10 ml) extrahiert. Die organischen Phasen wurden vereinigt, gewaschen, getrocknet, filtriert und im Vakuum aufkonzentriert. Der Rückstand wurde mittels Säulenchromatographie gereinigt (80:20 bis 75:25, Petrolether:Ethylacetat). Ausbeute an (2*S*, 4*S*, 5*S*)-5-Acetoxy-2-(*E*-hept-1-enyl)-3-hydroxy-oxolan (I) als farbloses Öl: 60% (42 mg, 0.174 mmol).
   ¹H NMR (CDCl₃, 200MHz): δ (ppm) 0.85 (t, 3H, *J* = 6.59 Hz), 1.22-1.50 (m, 6H), 1.81 (bs, 1H), 2.00-2.14 (m und Überlappungen s, 5H), 2.35 (ddq, 2H, *J* = 12.51, 5.86 und 2.19 Hz), 4.30-4.36 (m, 1H), 4.55 (dd, 1H, *J* = 6.35 und 2.69 Hz), 5.51 (ddt, 1H, *J* = 15.63, 6.60 und 1.41 Hz), 5.91 (dt, 1H, *J* = 15.57 und 6.59 Hz), 6.42 (dd, 1H, *J* = 5.86 und 2.25 Hz); ¹³C NMR (CDCl₃, 200MHz): δ (ppm), 14.03, 21.32, 22.51, 28.64, 31.43, 32.54, 41.98, 72.42, 83.62, 97.64, 122.93, 137.43, 170.38.
e) Zu einer gekühlten Lösung (5°C) von 42 mg der Verbindung I (0.176 mmol) in 0.5 ml CH2Cl2 wurden 42 mg Pyridin (0.53 mmol), gefolgt von 74 mg Trifluoressigsäureanhydrid (0.352 mmol) in einer Portion und unter Rühren zugesetzt. Nach weiteren 45 Minuten Rühren wurden 114 mg Natriumazid (1.76 mmol), gefolgt von 1 ml DMF zugegeben, wobei sich das Reaktionsgemisch auf Raumtemperatur erwärmte, bei der es über Nacht weitergerührt wurde. Nach Zugabe von 10 ml CH₂Cl₂ wurde die organische Phase mit Wasser (3 x 5 ml) gewaschen, gewaschen, getrocknet, filtriert und im Vakuum aufkonzentriert. Der Rückstand wurde mittels Säulenchromatographie gereinigt (90:10 bis 70:30, Petrolether:Ethylacetat). Ausbeute an (2*S*, 4*S*, 5*S*)-5-Acetoxy-3-azido-2-(*E*-hept-1-enyl)-oxolan (J) als farbloses Öl: 49% (24 mg; 0.085 mmol) und 24% (10 mg; 0.041 mmol) wiedergewonnene Ausgangsverbindung I.
   ¹H NMR (CDCl₃, 200MHz): δ (ppm) 0.88 (t, 3H, *J* = 6.59 Hz), 1.22-1.45 (m, 6H), 1.98-2.20 (m und Überlappungen s, 6H), 2.60 (ddd, 1H, *J* = 14.54, 8.60 und 5.62 Hz), 3.73 (dt, 1H, *J* = 10.55, 8.79 und 5.13 Hz), 4.47 (t, 1H, *J* = 6.59 Hz), 5.40 (ddt, 1H, *J* = 15.30, 7.33 und 1.28 Hz), 5.91 (dt, 1H, *J* = 15.38 und 6.64 Hz), 6.31 (dd, 1H, *J* = 5.67 und 1.81 Hz); ¹³C NMR (CDCl₃, 200MHz): δ (ppm), 14.06, 21.32, 22.52, 28.45, 31.36, 32.26, 37.71, 63.64, 84.75, 97.06, 126.12, 136.80, 170.29.
f) Aus J wurde durch Umsetzung mit Ozon und Reduktion analog zu Hoffman, J. Org. Chem. 1997 (62), 2458-2465 oder Hudlicky, , J. Org. Chem. 1998 (63) 510-520 die Endverbindung (2*R*, 4*S*, 5*S*)-5-Acetoxy-3-azido-2-hydroxymethyl-oxolan (K) hergestellt.

## Patentansprüche

1. Verfahren zur stereoselektiven Reduktion von chiralen α- oder β-Ketoestern, dadurch gekennzeichnet, dass α- oder β-Ketoester, die in γ-Position ein chirales Zentrum besitzen, in einem inerten Lösungsmittel bei Temperaturen von - 80 bis +50°C mit einem Reduktionsmittel, erhalten durch Reaktion von NaBH₄ und (*D*)- oder (*L*)-Weinsäure, zu den jeweils entsprechenden diastereomeren Hydroxyverbindungen reduziert werden.

2. Verfahren nach Anspruch 1, dadurch gekennzeichnet, dass als Substrat Verbindungen mit einer offenkettige, gegebenenfalls verzweigten Alkyl- oder Alkenylkette mit 4 bis 30, C-Atomen, wobei im Falle einer Alkenylkette, diese 1 bis 3 Doppelbindungen aufweisen kann, die in Position 1 mit einer Carboxylatgruppe mit 1 bis 20 C-Atomen im Esterteil substituiert ist und entweder in α- oder β-Position zur Carboxylatgruppe eine Ketogruppe und in γ-Position ein chirales Zentrum aufweist.

3. Verfahren nach Anspruch 1, dadurch gekennzeichnet, dass als Substrat (4S)-Methyl-3-oxo-4-tert-butylsilyloxy-undec-5-enoat eingesetzt wird.

4. Verfahren nach Anspruch 1, dadurch gekennzeichnet, dass das Reduktionsmittel in situ durch Reaktion von NaBH₄ und (*D*)- oder (*L*)-Weinsäure hergestellt wird.

5. Verfahren nach Anspruch 1, dadurch gekennzeichnet, dass als Lösungsmittel 2-Propanol, t-Butanol, Tetrahydrofuran, Dioxan, Diethylether, Benzol, Toluol oder Xylol oder ein Gemische daraus eingesetzt werden.

6. Verfahren nach Anspruch 3, dadurch gekennzeichnet, dass (4S)-Methyl-3-oxo-4-tert-butylsilyloxy-undec-5-enoat in Abhängigkeit von der eingesetzten Weinsäure zu (3*R*,4*S*)- oder (3*S*,4*S*)-Methyl-3-oxo-4-tert-butylsilyloxy-undec-5-enoat in einer optischen Reinheit von über 90% d.e. reduziert wird.

7. Verwendung von (3*R*,4*S*)- oder (3*S*,4*S*)-Methyl-3-oxo-4-tert-butylsilyloxy-undec-5-enoat, hergestellt nach Anspruch 1, zur Herstellung der entsprechenden enantiomeren- und diastereomerenreinen Lactone( 4*R*, 5*S*)-5-Hydroxymethyl-4-tetradecanoyl-tetrahydro-furan-2-on oder (*E*,4*S*,5*S*)-4-Hydroxy-5-(1-heptenyl)dihydro-furan-2-on, sowie dessen weitere Umsetzung zu dem L-AZT-Intermediat (*E*,2*S*,4*S*,5*S*)-2-O-Acetyl-4-azido-5-hydroxymethyl-dihydrofuran.

8. Verwendung von (3*R*, 4*S*)- Methyl-3-hydroxy-4-tert-butylsilyloxy-undec-5-enoat, hergestellt nach Anspruch 1, zur Herstellung von(4*R*, 5*S*)-5-Hydroxymethyl-4-tetradecanoyl-tetrahydro-furan-2-on, dadurch gekennzeichnet, dass
a) (3*R*, 4*S*)- Methyl-3-hydroxy-4-tert-butylsilyloxy-undec-5-enoat mit Tetrabutylammoniumfluorid in einem unter den Reaktionsbedingungen inerten Lösungsmittel bei 10 bis 40°C umgesetzt wird und Isolierung des Zwischenproduktes (4*R*, 5*S*)-5-(*E*-Hept-1-enyl)-4-hydroxy-tetrahydro-furan-2-on durch Extraktion, sodann
b) Umsetzung des Zwischenproduktes aus a) mit einem Gemisch aus Pyridin und Myristoylchlorid in CH₂Cl₂ bei 10 bis 40°C und Isolierung des Zwischenproduktes (4*R*, 5*S*)-5-(*E*-Hept-1-enyl)-4-tetradecanoyl-tetrahydro-furan-2-on durch Extraktion, und anschließend
c) Umsetzung des Zwischenproduktes aus b) mit Ozon bei -80 bis - 60°C, erwärmen auf 10 bis 40°C und Isolierung des Endproduktes (4*R*, 5*S*)-5-Hydroxymethyl-4-Tetradecanoyl-tetrahydro-furan-2-on durch Zusatz von BH₃:Me₂S unter Edelgasatmosphäre, sowie von MeOH nach 10 bis 30h.

9. Verwendung von (3*S*, 4*S*)- Methyl-3-hydroxy-4-tert-butylsilyloxy-undec-5-enoat zur Herstellung von (2*R*, 4*S*, 5*S*)-5-Acetoxy-3-azido-2-hydroxymethyl-oxolan, dadurch gekennzeichnet, dass
a) (3*S*, 4*S*)- Methyl-3-hydroxy-4-tert-butylsilyloxy-undec-5-enoat mit TBAF in einem unter den Reaktionsbedingungen inerten Lösungsmittel, bei 10 bis 40°C umgesetzt wird und Isolierung des Zwischenproduktes (4*S*, 5*S*)-5-(*E*-Hept-1-enyl)-4-hydroxy-tetrahydro-furan-2-on, sodann
b) Umsetzung des Zwischenproduktes aus a) mit einer gekühlten Lösung von Imidazol in DMF und t-Butyl-diphenylsilylchlorid, erwärmen auf 10 bis 40°C und Isolierung des Zwischenproduktes (4*S*, 5*S*)- 4-*tert*-butyldiphenylsilyloxy-5-(*E*-Hept-1-enyl)-tetrahydrofuran-2-on durch Extraktion, weiters
c) Umsetzung des Zwischenproduktes aus b) mit Di-iso-butyl-aluminiumhydrid unter Edelgasatmosphäre bei -80 bis -60°C, erwärmen auf 10 bis 40°C und Isolierung des Zwischenproduktes (2*S*, 4*S*, 5*S*)-5-Acetoxy-3-*tert*-butyldiphenylsilyloxy-2-(*E*-hept-1-enyl)-oxolan durch Extraktion, weiters
d) Umsetzung einer gekühlten Lösung von ((2*S*, 4*S*, 5*S*)-5-Acetoxy-3-*tert*-butyldiphenylsilyloxy-2-(*E*-hept-1-enyl)-oxolan mit TBAF, erwärmen auf 10 bis 40°C und Isolierung des Zwischenproduktes (2*S*, 4*S*, 5*S*)-5-Acetoxy-2-(*E*-hept-1-enyl)-3-hydroxy-oxolan durch Extraktion, sowie
e) Umsetzung einer gekühlten Lösung von (2S, 4S, 5S)-5-Acetoxy-2-(*E*-hept-1-enyl)-3-hydroxy-oxolan mit Pyridin und mit Trifluoressigsäureanhydrid, Zugabe von Natriumazid, gefolgt von DMF, erwärmen auf 10 bis 40°C und Isolierung des Zwischenproduktes (2*S*, 4*S*, 5*S*)-5-Acetoxy-3-azido-2-(*E*-hept-1-enyl)-oxolan durch Extraktion und
f) Umsetzung des Zwischenproduktes aus e) mit Ozon, anschließender Reduktion und Isolierung des Endproduktes (2*R*, 4*S*, 5*S*)-5-Acetoxy-3-azido-2-hydroxymethyl-oxolan.
